# EUROPÄISCHE PATENTANMELDUNG

(11) **EP 4 814 040 A1**
(43) Veröffentlichungstag der Anmeldung: **30.09.2026**
(21) Anmeldenummer: 25166097.3
(22) Anmeldetag: 25.03.2025
(51) Int. Cl.: C08J 3/20, C12N 11/08, C12N 11/14, C12N 11/18, B09B 3/60, C08L 101/16

(54) **BIOLOGISCH ABBAUBARE POLYESTERPOLYURETHANE DURCH ENZYMEINBAU**

(71) Anmelder: Covestro Deutschland AG, 51373 Leverkusen (DE); Rheinisch-Westfälische Technische Hochschule (RWTH) Aachen Körperschaft des öffentlichen Rechts, 52062 Aachen (DE)
(72) Erfinder: KNOPP, Daniela, 40223 Düsseldorf (DE); REISKY, Lukas, 50996 Köln (DE); SCHWANEBERG, Ulrich, 52056 Aachen (DE); JAEGER, Gernot, 51503 Rösrath (DE); GOEBBELS, Simone, 41352 Korschenbroich (DE); KESSLER, Michael, 51377 Leverkusen (DE); HAETTIG, Juergen, 51519 Odenthal (DE); FISCHER, Anne, 53947 Nettersheim (DE); SCHMIDT, Gudrun, 51381 Leverkusen (DE)
(74) Vertreter: Levpat

(57) **Zusammenfassung**

Die vorliegende Erfindung betrifft Polyesterpolyurethane, die aktive Esterasen enthalten. Diese Polymere zeichnen sich durch besonders gute biologische Abbaubarkeit unter Umweltbedingungen aus.

## Beschreibung

Die vorliegende Erfindung betrifft Polyesterpolyurethane, die aktive Esterasen enthalten. Diese Polymere zeichnen sich durch besonders gute biologische Abbaubarkeit unter Umweltbedingungen aus.

Polyesterpolyurethane sind in vielen Einsatzgebieten genutzte Polymere. In vielen Fällen ist es unvermeidlich, dass die daraus hergestellten Produkte oder Teile davon in die Umwelt freigesetzt werden, anstatt sachgerecht entsorgt zu werden. Wegen der hohen Persistenz dieser Werkstoffe kann das ein Problem sein.

Deswegen wäre es wünschenswert, die biologische Abbaubarkeit konventioneller Polyesterpolurethane zu verbessern. Entsprechend modifizierte Polyurethane würden sich nach ihrer Freisetzung nicht in der Umwelt anreichern. Ein Faktor, der die biologische Abbaubarkeit von Polyesterpolyurethanen limitiert, ist ihr Anteil an Urethangruppen, die innerhalb des Polymers kristalline Domänen bilden. Es ist bekannt, dass die Anwesenheit derartiger kristalliner Domänen den enzymatischen Abbau von Polyestern hemmt (Thomsen et. al (2024) "Enzymatic degradation of pol(ethylene terephthalate) (PET): Identifying the cause of the hypersensitive enzyme kinetic response to increased PET crystallinity", Enzyme and Microbial Technology, 173: 110353). Da der biologische Abbau von Polymeren in der Umwelt häufig enzymbasiert ist, ist dies ein Problem.

Die der vorliegenden Erfindung zugrundeliegende Studie konnte überraschend zeigen, dass die Einarbeitung von Esterasen in Polyesterpolyurethane dazu führt, dass diese Polymere zumindest teilweise abgebaut werden können, wenn sie unter Bedingungen inkubiert werden, die eine Aktivität der eingearbeiteten Enzyme ermöglichen. Modellversuche haben gezeigt, dass modifizierte Polyesterpolyurethane, die Esterasen enthalten, welche eine teilweise Depolymerisation des Polyesterpolyurethans ermöglichen, unter Umweltbedingungen erheblich besser abbaubar sind als Polyesterpolyurethane ohne Esterasen oder mit inaktiven Esterasen.

Somit ergibt sich die Möglichkeit, konventionelle Polyesterpolyurethane so zu modifizieren, dass sie unter Umweltbedingungen eine erheblich geringe Persistenz aufweisen. Dies ist besonders vorteilhaft für Anwendungen, bei denen diese Werkstoffe gezielt in die Umwelt ausgebracht werden, z.B. im landwirtschaftlichen Bereich, oder wo die unbeabsichtigte Freisetzung nicht zuverlässig verhindert werden kann.

Deswegen offenbart die vorliegende Patentanmeldung in den Patentansprüchen und der nachfolgenden Beschreibung Polyesterpolyurethane mit verbesserter Bioabbaubarkeit.

In einer ersten Ausführungsform betrifft die vorliegende Erfindung eine Polyesterpolyurethanzusammensetzung enthaltend wenigstens ein Polyesterpolyurethan und
(a) wenigstens ein Enzym, das durch eine Aminosäuresequenz gemäß SEQ ID NO.: 1 definiert ist oder eine Variante des vorgenannten Enzyms, die durch eine Aminosäuresequenz mit wenigstens 85 % Identität zu den in SEQ ID NO.: 1 definierten konservierten Aminosäuren definiert ist; oder
(b) wenigstens ein Enzym, das durch eine Aminosäuresequenz gemäß SEQ ID NO.: 4 oder 8 definiert ist oder eine Variante eines der vorgenannten Enzyme, die durch eine Aminosäuresequenz mit wenigstens 64 % Identität zu SEQ ID NO.: 4 oder wenigstens 85 % Sequenzidentität zu SEQ ID NO.: 8 definiert ist.

Eine Polyesterpolyurethanzusammensetzung ist eine Zusammensetzung, die wenigstens ein Polyesterpolyurethan und wenigstens ein eingearbeitetes Enzym wie oben definiert enthält. Sie kann optional Additive oder weitere Polymere enthalten. Soweit die Polyesterpolyurethanzusammensetzung weitere Polymere enthält, ist es bevorzugt, dass sie bezogen auf das Gesamtgewicht aller in ihr enthaltenen Polymere zu wenigstens 80 Gew.-%, stärker bevorzugt zu wenigstens 90 Gew.-% und am stärksten bevorzugt zu wenigstens 95 Gew.-% aus einem oder mehreren Polyesterpolyurethanen besteht. Enzyme werden bei dieser Berechnung nicht als Polymere behandelt.

### Polyesterpolyurethan

Das Polyesterpolyurethan ist bevorzugt ein thermoplastisches Polyesterpolyurethan (TPU). Erfindungsgemäß geeignete TPUs werden durch Reaktion wenigstens einer Isocyanatkomponente mit wenigstens einem Polyesterpolyol erhalten.

Bevorzugt werden TPU aus Reaktionsgemischen erhalten, die bezogen auf ihr Gesamtgewicht zwischen 50 Gew.-% und 70 Gew.-% wenigstens ein Polyesterpolyol enthalten. Ein TPU im Sinne der vorliegenden Anmeldung kann neben dem Polyesterpolyol weitere Polyole enthalten, insbesondere Polycarbonatpolyole und/oder Polyetherpolyole. Diese weiteren Polyole haben aber vorzugsweise einen Gewichtsanteil am Gesamtgewicht des Reaktionsgemisches, der maximal 20 Gew.-% nicht übersteigt.

Als "Isocyanatkomponente" wird die Gesamtheit aller Verbindungen bezeichnet, die zur Synthese eines TPU verwendet werden und wenigstens eine Isocyanatgruppe pro Molekül enthalten. Besonders bevorzugt enthält die Isocyanatkomponente wenigstens eine Verbindung mit einer durchschnittlichen Funktionalität von wenigstens 2 Isocyanatgruppen pro Molekül, nachfolgend auch als "Polyisocyanat" bezeichnet. Geeignete Polyisocyanate können aliphatisch, cycloaliphatisch oder aromatisch gebundene Isocyanatgruppen enthalten.

Geeignete Polyisocyanate mit aromatisch gebundenen Isocyanatgruppen sind 2,4- und 2,6-Diisocyanattoluol (TDI), 2,4'-, 4,4'-Diisocyanatodiphenylmethan (MDI) sowie deren mehrkernige Homologe und 1,5-Diisocyanatonaphthalin und die auf den vorgenannten monomeren Polyisocyanaten basierenden oligomeren Polyisocyanate.

Geeignete Polyisocyanate mit aliphatisch gebundenen Isocyanatgruppen sind 1,4-Diisocyanatobutan (BDI), 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan und die auf den vorgenannten monomeren Polyisocyanaten basierenden oligomeren Polyisocyanate.

Geeignete Polyisocyanate mit cycloaliphatisch gebundenen Isocyanatgruppen sind 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethyl-cyclohexan Isophorondiisocyanat; (IPDI), 1-Isocyanato-1-methyl-4(3)-isocyanatomethylcyclohexan, 2,4'- und 4,4'-Diisocyanatodicyclohexylmethan(H12MDI), 1,3-und 1,4-Bis(isocyanatomethyl)cyclohexan, Bis-(isocyanatomethyl)-norbornan (NBDI), 4,4'-Diisocyanato-3,3'-dimethyldicyclohexylmethan, 4,4'-Diisocyanato-3,3',5,5'-tetramethyl-dicyclohexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanato-adamantan, 1,3-Dimethyl-5,7-diisocyanatoadamantan und die auf den vorgenannten Diisocyanaten basierenden oligomeren Polyisocyanate.

Der Begriff "oligomere Polyisocyanate" bezeichnet Reaktionsprodukte, die durch Vernetzung von wenigstens zwei monomeren Isocyanaten unter Ausbildung wenigstens einer Uretdion-, Isocyanurat-, Allophanat-, Biuret-, Iminooxadiazindion- und/oder Oxadiazintrionstruktur erhalten werden. Vorzugsweise enthalten oligomere Polyisocyanate zwei bis vier Moleküle eines monomeren Polyisocyanats.

Die verwendeten Polyesterpolyole sind vorzugsweise aus Dicarbonsäuren mit 2 bis 12, bevorzugt 4 bis 6 Kohlenstoffatomen und mehrwertigen Alkoholen aufgebaut. Bevorzugte Dicarbonsäuren sind Bernsteinsäure, Adipinsäure und Glutarsäure. Es ist auch möglich, Gemische verschiedener Dicarbonsäuren einzusetzen. Anstelle der Dicarbonsäuren können auch Dicarbonsäureester zur Synthese verwendet werden. Diese haben vorzugsweise 1 bis 4 Kohlenstoffatome im Alkoholrest. Alternativ können auch Carbonsäureanhydride oder Carbonsäurechloride zur Synthese eingesetzt werden. Bevorzugte mehrwertige Alkohole sind Glykole mit 2 bis 10, vorzugsweise 2 bis 6 Kohlenstoffatomen, insbesondere Ethylenglykol, Diethylenglykol, 1,4-Butandiol, 1,5-Pentandiol, 1,6-Hexandiol, 1, 10-Decandiol, 2,2-Dimethyl-1,3-propandiol, 1,3-Propandiol und Dipropylenglykol. Je nach den gewünschten Eigenschaften des TPU können die mehrwertigen Alkohole allein oder gegebenenfalls in Mischung untereinander verwendet werden. Geeignet sind ferner Ester der Kohlensäure mit den genannten Diolen, insbesondere solchen mit 4 bis 6 Kohlenstoffatomen, insbesondere 1,4-Butandiol oder 1,6-Hexandiol, Kondensationsprodukte von Hydroxycarbonsäuren, bevorzugt Hydroxycapronsäure und Polymerisationsprodukte von Lactonen, bevorzugt Caprolactonen. Caprolactone können ggf. substituiert sein.

Bevorzugte TPUs sind Ethandiol-polyadipate, 1,4-Butandiol-polyadipate, Ethandiol-1,4-butandiol-polyadipate, 1,6-Hexandiol-neopentylglykol-polyadipate, 1,6-Hexandiol-1,4-butandiol-polyadipate und Polycaprolactone.

Erfindungsgemäß besonders geeignete TPUs haben ein zahlenmittleres Molekulargewicht zwischen 500 und 4.000 g/mol. Besonders bevorzugt enthalten sie Polyesterpolyole, die 1,4-Butandiol enthalten. Ihre Polyisocyanatkomponente enthält besonders bevorzugt Methylen-Diphenyl-Diisocyanat (MDI) oder Hexamethylen-Diisocyanat (HDI).

### Enzyme

**Tabelle 1: Aminosäuresequenzen der genutzten Enzyme**

| Enzym | SEQ ID NO.: | Organismus |
|---|---|---|
| Konsensussequenz | 1 | |
| ThcCut1 | 2 | *Thermobifida cellulosilytica* DSM44535 |
| ThcCut1-ACCG | 3 | *Thermobifida cellulosilytica* DSM44535 |
| SvCut190*SS | 4 | *Saccharomonospora viridis* |
| HiC | 5 | *Thermomyces (Humicola) insolens* |
| PesH1 | 6 | Metagenomische DNA |
| TfCut2 | 7 | *Thermobifida fusca* KW3 |
| LCC | 8 | Metagenomische DNA |
| LCC-WCCG | 9 | Metagenomische DNA |
| LCC-ICCG | 10 | Metagenomische DNA |

Enzyme mit den an anderer Stelle in dieser Anmeldung definierten Sequenzidentitäten zu den durch SEQ ID NO.: 2 bis SEQ ID NO.: 10 definierten Enzymen bzw. zu der durch SEQ ID NO.: 1 definierten Konsensussequenz können durch Hinzufügung, Austausch oder Deletion von Aminosäuren erhalten werden. Solche Enzyme mit Aminosäuresequenzen die von SEQ ID NO.: 1 bis 10 abweichen, werden auch als "Varianten" bezeichnet". Jede erfindungsgemäße Variante ist vorzugsweise dadurch gekennzeichnet, dass sie Estergruppen in einem Polyesterpolyurethan spalten kann. Diese Enzymaktivität wird vorzugsweise durch eine Verringerung des pH-Wertes nachgewiesen, den die Spaltung der Polyeresterkomponente des Polyesterpolyurethans bewirkt. Dies kann mit allen geeigneten Verfahren erfolgen, wie sie z.B. in den Ausführungsbeispielen beschrieben sind. Besonders bevorzugt erfolgt sie mit dem chromatographischen Nachweis der nach Esterhydrolyse freigesetzten Monomere.

In einer bevorzugten Ausführungsform enthält die Polyesterpolyurethanzusammensetzung wenigstens ein Enzym, dessen Aminosäuresequenz wie folgt definiert ist: (i) Die zu den konservierten Aminosäurepositionen gemäß SEQ ID NO.: 1 homologen Aminosäurepositionen des Enzyms weisen wenigstens 90 %, stärker bevorzugt wenigstens 95 % Sequenzidentität zu den konservierten Aminosäurepositionen aus SEQ ID NO.: 1 auf und (ii) die zu den beliebigen Aminosäurepositionen aus SEQ ID NO.: 1 homologen Aminosäurepositionen weisen wenigstens 70 %, bevorzugt wenigstens 80 % Sequenzidentität zu den homologen Aminosäurepositionen einer der Aminosäuresequenzen ausgewählt aus der Gruppe bestehend aus SEQ ID NO.: 5, 2, 3, 4, 6, 7, 8, 9 und 10 auf. Bevorzugt weisen die beliebigen Aminosäurepositionen hierbei die vorgenannten Sequenzidentitäten zu SEQ ID NO.: 4 oder 8 auf.

Mit anderen Worten ist das Enzym in dieser Ausführungsform dadurch gekennzeichnet, dass seine funktionell bedeutsamen Aminosäurepositionen eine hohe Übereinstimmung mit der Konsensussequenz aufweisen, während die nicht konservierten und funktionell weniger relevanten Positionen mit größerer Sequenzabweichung von einem der in der dieser Anmeldung zugrunde liegenden Studie als aktiv identifizierten Enzymen abgeleitet ist.

In einer bevorzugten Ausführungsform der vorliegenden Erfindung ist die weiter oben definierte Variante eines durch SEQ ID NO.: 4 definierten Enzyms durch eine Sequenzidentität von wenigstens 74 %, bevorzugt wenigstens 80 %, stärker bevorzugt wenigstens 85 %, noch stärker bevorzugt wenigstens 90 % und am stärksten bevorzugt wenigstens 95 % mit der durch SEQ ID NO: 4 definierten Aminosäuresequenz gekennzeichnet.

In einer weiteren bevorzugten Ausführungsform der vorliegenden Erfindung ist die weiter oben definierte Variante eines durch SEQ ID NO.: 8 definierten Enzyms durch eine Sequenzidentität von wenigstens 90 % und bevorzugt wenigstens 95 % mit der durch SEQ ID NO. 8 definierten Aminosäuresequenz gekennzeichnet.

In noch einer weiteren Ausführungsform enthält die Polyesterpolyurethanzusammensetzung wenigstens ein Enzym, das eine Aminosäuresequenz hat wie in SEQ ID NO.: 5, 2, 3, 4, 6, 7, 8, 9 oder 10 definiert oder eine Variante eines der vorgenannten Enzyme mit wenigstens 85 % Sequenzidentität zu einer der vorgenannten Sequenzen.

In einer bevorzugten Ausführungsform enthält die Polyesterpolyurethanzusammensetzung wenigstens ein Enzym, das eine Aminosäuresequenz hat wie in SEQ ID NO.: 5, 3, 4 oder 10 definiert oder eine Variante eines der vorgenannten Enzyme mit wenigstens 85 % Sequenzidentität zu einer der vorgenannten Sequenzen.

In einer besonders bevorzugten Ausführungsform enthält die Polyesterpolyurethanzusammensetzung wenigstens ein Enzym, das eine Aminosäuresequenz hat wie in SEQ ID NO.: 5 definiert oder eine Variante dieses Enzyms mit wenigstens 85 % Sequenzidentität zu der vorgenannten Sequenz.

Eine Variante wird aus den erfindungsgemäßen Aminosäuresequenzen bevorzugt durch Hinzufügung, Deletion oder Austausch des jeweils definierten Anteils der im jeweiligen Enzym enthaltenen Aminosäuren erhalten. Basis für die Berechnung der Sequenzidentität ist bevorzugt jeweils die durch SEQ ID NO.: 2, 3, 4, 5, 6, 7, 8, 9 oder 10 definierte Aminosäuresequenz. Dem Fachmann ist bewusst, dass Fusionsproteine von Enzymen mit anderen Proteinen gebildet werden, ohne dass dies die Aktivität des Enzyms beeinträchtigt. Deswegen umfasst der Begriff "Variante" auch solche von den durch SEQ ID NO.: 2, 3, 4, 5, 6, 7, 8, 9 oder 10 definierten Polypeptiden abgeleiteten Aminosäuresequenzen, die am N-Terminus und/oder am C-Terminus mit weiteren Proteinen, z.B. dem Green Fluorescent Protein, fusioniert sind.

Besonders bevorzugte Varianten der erfindungsgemäßen Enzyme werden durch Hinzufügung, Deletion oder Austausch von bis zu 20, bevorzugt bis zu 10 und noch stärker bevorzugt bis zu 5 Aminosäuren der offenbarten Sequenzen erhalten. Grundsätzlich können die vorgenannten Modifikationen an jeder beliebigen Stelle des betreffenden Polypeptids kontinuierlich oder diskontinuierlich erfolgen. Bevorzugt erfolgen sie aber nur am N-Terminus und/oder am C-Terminus des Polypeptids.

In einer bevorzugten Ausführungsform der vorliegenden Erfindung enthält die Polyesterpolyurethanzusammensetzung mehr als ein Enzym, insbesondere Mischungen von wenigstens zwei oder drei unterschiedlichen Enzymen. Hierdurch ist es möglich, synergistische Effekte von Enzymen mit unterschiedlichen Aktivitätsoptima oder unterschiedlicher Substratspezifizität auszunutzen.

Die erfindungsgemäße Polyesterpolyurethanzusammensetzung enthält bezogen auf das Gesamtgewicht der in ihr enthaltenen Polyesterpolyurethane zwischen 0,1 Gew-% und 2 Gew.-% des Enzyms und der Enzyme. Stärker bevorzugt sind 0,1 Gew.-% bis 1,0 Gew.-%.

Die erfindungsgemäßen Polyesterpolyurethanzusammensetzungen zeichnen sich durch eine verbesserte biologische Abbaubarkeit aus. Unter "biologischer Abbaubarkeit" wird hier die Zersetzbarkeit der in der Polyesterpolyurethanzusammensetzungen enthaltenen Polyesterpolyurethane durch Mikroorganismen verstanden. Die Produkte der Zersetzung sind vorzugsweise mikrobielle Biomasse, Kohlendioxyd und Wasser.

In einer weiteren bevorzugten Ausführungsform der vorliegenden Anmeldung enthält die Polyesterpolyurethanzusammensetzung wenigstens einen Säurefänger. Bevorzugt ist der Säurefänger ein anorganisches Salz ausgewählt aus der Gruppe bestehend aus Carbonaten, Hydrogencarbonaten, Phosphaten und Hydroxiden. Bevorzugte Carbonate sind Natriumcarbonat, Kaliumcarbonat und Calciumcarbonat. Bevorzugte Hydrogencarbonate sind Natriumhydrogencarbonat, Kaliumhydrogencarbonat und Calciumhydrogencarbonat. Bevorzugte Phosphate sind Natriumphosphat, Dinatriumhydrogenphosphat und Natriumdihydrogenphosphat. Ein bevorzugtes Hydroxid ist Magnesiumhydroxid. Säurefänger, insbesondere die vorgenannten anorganischen Salze, haben vorzugsweise einen Gewichtsanteil zwischen 5 Gew.-% und 25 Gew.-% an der Polyesterpolyurethanzusammensetzung. Diese Werte sind bezogen auf das Gesamtgewicht der in der Polyesterpolyurethanzusammensetzung enthaltenen Polyesterpolyurethane.

In noch einer weiteren Ausführungsform betrifft die vorliegende Anmeldung die Verwendung eines Enzyms, das
(i) durch eine Aminosäuresequenz gemäß SEQ ID NO.: 1 definiert ist oder eine Variante des vorgenannten Enzyms, die durch eine Aminosäuresequenz mit wenigstens 85 % Identität zu den in SEQ ID NO.: 1 definierten konservierten Aminosäuren definiert ist; oder
(ii) durch eine Aminosäuresequenz gemäß SEQ ID NO.: 4 oder 8 definiert ist oder eine Variante eines der vorgenannten Enzyme, die durch eine Aminosäuresequenz mit wenigstens 64 % Identität zu SEQ ID NO.: 4 oder wenigstens 85 % Sequenzidentität zu SEQ ID NO.: 8 definiert ist;
zur Verbesserung der biologischen Abbaubarkeit eine Polyesterpolyurethans.

Alle weiter oben in dieser Anmeldung gegebenen Definitionen, insbesondere betreffend das Enzym und das Polyesterpolyurethan, gelten auch für diese Ausführungsform.

Die Verwendung des Enzyms besteht in der Einarbeitung des Enzyms in eine Zusammensetzung, die wenigstens ein Polyesterpolyurethan wie oben definiert enthält. Bevorzugt handelt es sich um eine Polyesterpolyurethanzusammensetzung, die in Polymerprodukten enthalten ist, die absichtlich in die Umwelt ausgebracht wird. Es kann sich aber auch um eine Polyesterpolyurethanzusammensetzung handeln, die in Polymerprodukten enthalten ist, bei denen nicht ausgeschlossen werden kann, dass sie in der Umwelt verbleiben, auch wenn dies nicht geplant ist. Letzteres gilt für Produkte, z.B. für Verpackungsmaterialien, die grundsätzlich einer regulären Abfallentsorgung zugeführt werden sollen, bei denen aber nicht auszuschließen ist, dass Teile davon in der Umwelt verbleiben, z.B. durch Unachtsamkeit. Unter "Umwelt" wird in dieser Anmeldung bevorzugt der Boden verstanden.

### Einarbeitung

Um eine Polyesterpolyurethanzusammensetzung zu erhalten, die wenigstens eines der erfindungsgemäß einsetzbaren Enzyme enthält, wird vorzugsweise das wenigstens eine Enzym in das vorher hergestellte Polymer eingearbeitet. Mit anderen Worten: Das Enzym wird vorzugsweise nicht schon während der Synthese des Polyesterpolyurethans in das Reaktionsgemisch eingebracht.

Zur Einarbeitung eines Enzyms in das fertige Polyesterpolyurethan gibt es grundsätzlich zwei bevorzugte Verfahren.

In einer ersten Ausführungsform wird wenigsten ein Polyesterpolyurethan in einem geeigneten Lösungsmittel gelöst und das wenigstens eine Enzym zum gelösten Polymer zugegeben. Durch Verdampfen des Lösungsmittels wird dann das Polyesterpolyurethan erhalten, das das wenigstens eine Enzym enthält. Dieses Verfahren ist günstig, weil es keine Temperaturbelastung der Enzyme mit sich bringt. Geeignete Lösungsmittel sind insbesondere Tetrahydrofuran (THF), Aceton, Methyethylketon, Toluol, Cyclohexanon, Trichlorethylen und N-Hexan. Hierbei ist THF besonders bevorzugt.

In einer anderen Ausführungsform wird wenigstens ein TPU aufgeschmolzen und das Enzym in die Schmelze gegeben. Das flüssige TPU kann dann durch alle dem Fachmann bekannten Verfahren in die gewünschte Form gebracht werden. Dies kann besonders vorteilhaft durch Gießen, insbesondere Spritzguss, oder durch Extrusion erfolgen. Nach seinem Erstarren liegt ein thermoplastisches Polyesterpolyurethan vor, das das wenigstens eine Enzym enthält. Da diese Form der Einarbeitung eine Erwärmung des TPU über den Schmelzpunkt erfordert, ist das Enzym ebenfalls den entsprechenden Temperaturen ausgesetzt. Deswegen ist es für diese Ausführungsform bevorzugt, thermostabile Enzyme zu verwenden, die auch nach der Behandlung mit den erforderlichen Temperaturen ihre Aktivität weitgehend behalten. Bevorzugte thermostabile Enzyme sind HiC (SEQ ID No. 5), ThcCut1-ACCG (SEQ ID NO. 3), SvCut190*SS (SEQ ID NO. 4) und LCC-ICCG (SEQ ID NO. 10)und deren oben definierte Varianten.

Die Einarbeitung der erfindungsgemäßen Enzyme in wenigstens ein Polyesterpolyurethan führt zu einer Polyesterpolyurethanzusammensetzung, die unter Umweltbedingungen um bis zu 30 % schneller abgebaut wird als eine Zusammensetzung ohne aktives Enzym. Somit besteht bei - beabsichtigter oder versehentlicher - Ausbringung der entsprechenden Polyesterpolyurethanzusammensetzungen in die Umwelt eine geringe Gefahr, dass sie dort akkumulieren und schädliche Wirkungen entfalten.

In einer weiteren Ausführungsform betrifft die vorliegende Erfindung ein Polymerprodukt, das die erfindungsgemäße Polyurethanzusammensetzung enthält oder aus ihr besteht. Das Polymerprodukt ist vorzugsweise ausgewählt aus der Gruppe bestehend aus Verpackungsmaterialien, Befestigungsmitteln für Pflanzen, insbesondere Rankhilfen, Kunststoffbestandteilen von Feuerwerkskörpern, Borsten von Besen, Markierungsbändern für Tiere und Abfallbeuteln, insbesondere für Biomüllwelches die erfindungsgemäße Polyesterpolyurethanzusammensetzung enthält.

### Verfahren

In einer weiteren Ausführungsform betrifft die vorliegende Erfindung ein Verfahren zur Herstellung einer Polyesterpolyurethanzusammensetzung enthaltend die die Schritte
a) Bereitstellung wenigstens eines Polyesterpolyurethans und wenigstens eines Enzyms, das
   (i) durch eine Aminosäuresequenz gemäß SEQ ID NO.: 1 definiert ist oder eine Variante des vorgenannten Enzyms, die durch eine Aminosäuresequenz mit wenigstens 85 % Identität zu den in SEQ ID NO.: 1 definierten konservierten Aminosäuren definiert ist; oder
   (ii) durch eine Aminosäuresequenz gemäß SEQ ID NO.: 4 oder 8 definiert ist oder eine Variante eines der vorgenannten Enzyme, die durch eine Aminosäuresequenz mit wenigstens 64 % Identität zu SEQ ID NO.: 4 oder wenigstens 85 % Sequenzidentität zu SEQ ID NO.: 8 definiert ist;
b) Vermischen des wenigstens einen Polyesterpolurethans mit dem wenigstens einen Enzym, wobei das wenigstens eine Polyesterpolyurethan entweder als Schmelze oder in einem geeigneten Lösungsmittel gelöst vorliegt; und
c) Aushärten der Polyesterpolyurethanzusammensetzung durch Abkühlung der Schmelze oder durch Entfernung des Lösungsmittels.

Alle oben gegeben Definitionen gelten auch für diese Ausführungsform.

In noch einer weiteren Ausführungsform betrifft die vorliegende Erfindung eine Polyesterpolyurethanzusammensetzung erhalten oder erhältlich durch das vorangehend definierte Verfahren.

Die nachfolgenden Ausführungsbeispiele dienen nur dazu, die Erfindung zu illustrieren. Sie sollen den Schutzbereich der Patentansprüche in keiner Weise beschränken.

### Beispiele

### Enzyme

Das kommerziell erhältliche Enzym, das vermutlich die Aminosäuresequenz gemäß SEQ ID NO.: 5 hat, wurde von Strem Chemicals (Bischheim, Frankreich) bezogen.

### Plasmid Konstruktion

Das Gen für die SvCut190*SS wurde von Eurofins Genomics (Ebersberg, Deutschland) synthetisiert und mittels Restriktionsklonierung mit Xhol und Ndel in den pET26b(+)-Vektor von Merck (Darmstadt, Deutschland) kloniert. Die Gene für die LCC-ICCG und die ThcCut1 wurden ebenfalls über die Restriktionsklonierung mit Xhol und Ndel in den pET26b(+)-Vektor von Merck (Darmstadt, Deutschland) kloniert. Die ThcCut1-ACCG wurde basierend auf dem pET26b(+)-Vektor enthaltenen ThcCut1 mittels Mutation-PCR hergestellt. Für die Vermehrung der Plasmide wurde eine Transformation chemisch kompetenter *E. coli* DH5α-Zellen durchgeführt. Gewachsene Einzelkolonien wurden zur Inokulation von 4,5 ml LB-Medium mit 50 µg/ml Kanamycin verwendet und entweder in 20 % Glycerol-Stocks konserviert oder gemäß den Herstellerangaben für die Sequenzierung bei GeneWiz (Leipzig, Deutschland) vorbereitet.

### Heterologe Proteinexpression

Die heterologe Expression der Enzyme erfolgte in mit den Plasmid-Konstrukt transformierten *E. coli* BL21 (DE3)-Zellen. 4,5 ml LB-Medium mit 1 % (w/v) Glucose und 50 µg/ml Kanamycin wurden aus einer Glycerol-Stammkultur beimpft und über Nacht bei 37 °C und 200 rpm inkubiert. 200 ml ZYP-5052-Autoinduktionsmedium mit 50 µg/ml Kanamycin wurden mit 0,2 % frisch gewachsenen Zellen beimpft und bei 37 °C und 200 rpm für 4 Std. in Kolben inkubiert. Die Expression erfolgte für 24 Stunden bei 20°C und 200 rpm. Die Zellen wurden bei 4 °C für 20 min bei 8.000 x g geerntet.

### Zelllyse

Die geernteten Zellen wurden in 10 ml 20 mM KPi-Puffer pH 7,5 resuspendiert. Die Zellen wurden dreimal mit Ultraschall (Bandelin Sonoplus HD, Sonotrode MS73, Berlin, Deutschland) unter konstanter Kühlung in einem Eisbad für 2 min (Amplitude: 50 %) aufgeschlossen. Die resultierende Suspension wurde bei 10.000 x g und 4 °C für 40 min zentrifugiert. Der Überstand wurde mit einem 0,2 µm PES-Filter filtriert.

### Lyophilisierung des zellfreien Expressionskulturüberstands

Der filtrierte Überstand der lysierten Zellen wurde bei -80 °C eingefroren. Im Anschluss wurde der gefrorene zellfreie Überstand 3 Tage lang bei -65 °C Kühlertemperatur und 0,1 mbar lyophilisiert. Das Enzymlyophilisat wurde bei -4°C gelagert.

### Herstellungvon Novozym^{®} 51032 Lyophilisat

Das kommerziell erhältliche Flüssigpräparat Novozym^{®} 51032 wurde über Crossflow Filtration mit der Vivaflow 50 MWCO 10000 Kassette umgepuffert, um das Glycerol der Stock-Lösung durch Ammoniumacetatpuffer (20 mM) auszutauschen. Das umgepufferte Enzym wurde im Anschluss für 3 Tage bei -65 °C Kühlertemperatur und 0,1 mbar lyophilisiert. Das so erhaltene Enzymlyophilisat wurde bei 4°C gelagert.

### Natriumdodecylsulfat-Polyacrylamidgelelektrophorese (SDS-PAGE)

Die Enzymexpression und Proteinreinheit wurden mittels Natriumdodecylsulfat-Polyacrylamidgelelektrophorese (SDS-PAGE) untersucht. Dafür wurde ein Kulturvolumen äquivalent zu 1 ml mit einer optischen Dichte von 5 (bei 600 nm) entnommen und bei 13.000 rpm für 2 min zentrifugiert. Der Überstand wurde verworfen und das Zellpellet wurde in 200 µl 1 x PBS Puffer resuspendiert. Zu jedem Zellpellet wurden 200 µl BugBuster^{®} Proteinextraktionsreagenz versetzt mit 1 µl/ml Benzonase gegeben. Für die Zelllyse inkubierten die Proben 20 min bei Raumtemperatur unter gelegentlichem Invertieren. Nach der Lyse wurden 14 µl der lysierten Zellsuspension, mit 7 µl 3x Reducing Blue Protein Loading Dye gemischt (= Ganzzellextrakt). Der Rest der lysierten Zellsuspension wurde für 5 min bei 13.000 rpm zentrifugiert. Von dem resultierenden Überstand wurden wiederum 14 µl mit 7 µl 3x Reducing Blue Protein Loading Dye gemischt (= lösliche Enzymfraktion). Beide Ansätze wurden 5 min bei 95 °C inkubiert. 15 µl der Proben wurden zusammen mit 3 µl EZ-Run^{®} Prestained Rec Protein Ladder auf ein vorgegossenes NuPAGE 4- 12% Bis-Tris-Gel aufgetragen. Die Proteine wurden bei konstanter Spannung von 200 V für 40 min elektrophoretisch aufgetrennt. Die Gele wurden 2 Std. mit 30-50 ml Roti BlueQuick gebrauchsfertiger Färbelösung angefärbt und anschließend 30-60 min mit Reinstwasser entfärbt.

### Bestimmung der Proteinkonzentration

Die Proteinkonzentration wurde kolorimetrisch mit dem Bradford-Assay bestimmt. Dazu wurde eine Rinderserumalbumin (BSA)-Standardkurve von 125-1.000 µg/ml hergestellt. Die lyophilisierten Enzyme und die Leervektorkontrolle wurden in Reinstwasser gelöst und bei Bedarf ebenfalls mit Reinstwasser verdünnt. Zu 5 µl BSA- und Enzymlösung wurden 250 µl Quick Start Bradford Reagenz, 1x, hinzugegeben. Nach 5 min Inkubation wurde die Absorption bei 595 nm gemessen.

### Test der Enzymaktivität mit p-Nitrophenylbutyrat (pNPB)

Die Enzymaktivität und die der Leervektorkontrolle wurde mit dem Esterhydrolyse-Substrat p-Nitrophenylbutyrat (pNPB) bestimmt, wobei dessen Hydrolyse zur Freisetzung von p-Nitrophenol (pNP) führt, was photometrisch gemessen wird. Dazu wurde eine pNP-Standardkurve von 0,001-0,05 mg/ml hergestellt und die Absorption bei 410 nm gemessen (pNP, Sigma Aldrich). Für die Enzymaktivitätsmessung wurden die lyophilisierten Enzyme in Reinstwasser gelöst und bei Bedarf ebenfalls mit Reinstwasser verdünnt. Die pNPB-Substratlösung wurde hergestellt, indem das kommerziell erhältliche pNPB-Präparat (Sigma Aldrich) mit DMSO auf eine Konzentration von 5 mg/ml eingestellt wurde. Im Anschluss erfolgte eine 1:10 Verdünnung der pNPB-Lösung mit KPi-Puffer (100 mM, pH 7,0) auf eine finale Konzentration von 0,5 mg/ml. Zu 20 µl Enzymlösung wurden 180 µl der 0,5 mg/ml pNPB-Lösung hinzugegeben. Die Absorption von pNP wurde bei 410 nm und 30°C über einen Zeitraum von 15 Minuten alle 15 Sekunden gemessen. Die Aktivität wurde aus der Anfangssteigung berechnet.

### Thermoplastische Polyesterpolyurethane

TPU 1 besteht aus 63,01 Gew.-% Polyesterpolyol (basierend auf Adipinsäure und 1,4-Butandiol) mit einem zahlenmittleren Molekulargewicht von 2250 g/mol, sowie 1,4-Butandiol und MDI.

TPU 2 besteht aus 59,07 Gew.-% Polyesterpolyol (basierend auf Adipinsäure und 1,4-Butandiol) mit einem zahlenmittleren Molekulargewicht von 2250 g/mol, sowie 1,4-Butandiol, HDI und eine Laktid-basiertem Polyol.

### Einarbeitung der Enzyme per Schmelzextrusion

Für die Einarbeitung von Enzymen in TPUs wurde ein Doppelschnecken-Mico-Compounder, Modell Xplore MC 15 (DSM), verwendet. Das TPU-Granulat wurde im Falle des TPU 1 unter Zugabe von flüssigem Stickstoff zu einer Partikelgröße von ≤ 1mm gemahlen, wobei das TPU 2 ungemahlen mit einer Partikelgröße von 3-5 mm verwendet wurde. In beiden Fällen wurde das TPU vor Verwendung in der Extrusion 48 Stunden bei 70°C getrocknet. Einzuarbeitende Enzyme wurden als Lyophilisat für die Extrusion vorbereitet, wobei es sich um den zellfreien Überstand einer Expressionskultur handelt. Das kommerziell erhältliche Novozym^{®} 51032 Präparat wurde nach Umpuffern und Gefriertrocknen eingearbeitet. Für eine homogene Enzymverteilung wurde ein Premix aus TPU und Enzymlyophilisat hergestellt, der einen Enzymanteil von 0,3 Gew.-% bezogen auf das TPU-Gewicht enthielt. Für TPU 2 wurde zusätzlich ein Ansatz mit 20 Gew.-% Calciumcarbonat und 0,3 Gew.-% Enzymanteil bezogen auf das TPU-Gewicht hergestellt. Zudem wurden mit beiden TPUs Ansätze zum Spülen des Extruders zwischen jedem Probenwechsel, vorbereitet wobei ebenfalls TPU mit 0,3 Gew.-% Enzymlyophilisat gemischt wurde. Die Probenfüllzeit des Extruders betrug ≤ 1 Minute. Nachdem die gesamte Probe in den Mischbereich des Extruders gefüllt wurde, wurde das Compoundieren für 1:30 Minuten bei der eingestellten Schmelztemperatur (Tabelle 2) mit einer Rotationsgeschwindigkeit der beiden Extruderschnecken von 50 U/min durchgeführt, bevor das TPU in Form eines Strangs den Extruder verließ. Die Proben wurden bei Raumtemperatur abgekühlt und 24 Stunden bei 70°C getrocknet, bevor Abbaustudien durchgeführt wurden.

**Tabelle 2: Übersicht der Extrusionsbedingungen zur Einarbeitung der Enzyme in TPUs 1 und 2.**

| **Additiv** | pET26b_LV | Novozym^{®} 51032 | SvCut190*SS | ThcCut1-ACCG | LCC-ICCG |
|---|---|---|---|---|---|
| **TPU 1** | - | 210 | - | - | - |
| | - | 200 | - | - | - |
| | 195 | - | 195 | 195 | 195 |
| | - | 190 | 190 | | |
| **TPU 2** | - | 200 | 200 | 200 | 200 |
| | - | 190 | 190 | 190 | 190 |
| | - | 180 | 180 | 180 | 180 |
| | - | 170 | 170 | 170 | 170 |
| | - | 160 | 160 | 160 | 160 |
| | 150 | 150 | 150 | 150 | 150 |

### Einarbeitung der Enzyme mit lösemittelbasiertem Verfahren

Für die lösemittelbasierte Einarbeitung der Cutinasen Novozym^{®} 51032, ThcCut1, ThcCut1-ACCG, SvCut190*SS, LCC ICCG und der Leervektorkontrolle pET26b_LV in TPU 1 wurde eine 5 Gew.-% TPU-Lösung in THF hergestellt. Das TPU-Granulat wurde unter Rühren auf einem Magnetrührer für 48 Stunden gelöst. Nachdem das TPU nahezu vollständig gelöst war, wurden die Lyophilisate in Konzentrationen von 0,3 Gew.-% und 0,5 Gew.-% bezogen auf das Polymergewicht in geeignete Glasschalen gegeben, die entsprechende Menge TPU-Lösung hinzugefügt und alles durch Pipettieren vermischt.

Für die lösemittelbasierte Einarbeitung der Enzyme LCC, LCC-WCCG, TfCut2 und PesH1 in TPU 1, wurde eine 4 Gew.-% TPU-Lösung in THF hergestellt. Das TPU-Granulat wurden unter Rühren auf einem Magnetrührer für 24 Stunden gelöst. Nachdem das TPU vollständig gelöst war, wurden die Lyophilisate in Konzentrationen von 0,5 Gew.-% bezogen auf das Polymergewicht in geeignete Glasschalen gegeben, die entsprechende Menge TPU-Lösung hinzugefügt und alles durch Pipettieren vermischt.

Für die Negativkontrolle ohne Enzymzugabe wurde reine TPU-Lösung in eine Glasschale überführt. In allen Fällen wurden die Volumina so gewählt, dass die Filme dieselbe Schichtdicke aufwiesen. Das THF wurde für 2 Tage bei Raumtemperatur abgedampft, und die getrockneten Filme wurden für die Abbaustudien verwendet.

### PU-Abbaustudien unter Laborbedingungen im wässrigen System

Die TPU-Abbaustudien wurden standardmäßig mit 200 mg TPU-Probe durchgeführt. Die TPU-Proben (Film oder Strang) wurden über Nacht bei 70°C in einem Umluftofen getrocknet. Im Anschluss wurden die Proben in gleichgroße Stücke geschnitten und im Falle der TPU-Filme in 15 ml Zentrifugationsröhrchen oder im Falle der TPU-Stränge in die Wells einer Deep-Well Platte eingewogen. Die Abbaustudie von Proben mit eingearbeiteten Enzymen wurde durch Zugabe von 5 ml KPi-Puffer (200 mM, pH 8,0) gestartet. Die Kontrollreaktionen wurden durch externe Enzym- und ebenfalls KPi-Zugabe angesetzt, sodass ein finales Volumen von 5 ml nicht überschritten wurde. Für die externe Enzymzugabe wurden die jeweiligen Enzymlyophilisate und das Leer-Vektor-Lyophilisat in Reinstwasser gelöst, durch einen 0,2 µm PES-Filter steril filtriert und in Endkonzentrationen von 0,3 Gew.-% oder 0,5 Gew.-% zu jeder TPU-Probe gegeben. Für die Negativkontrollen wurde den Proben nur 5 ml KPi-Puffer zugesetzt. Alle Proben inkubierten bei 37°C über einen Zeitraum von mindestens 14, standardmäßig 21 und in einigen Fällen 33 Tagen bei 150-200 rpm. Für die Adipinsäurequantifizierung wurden zu bestimmten Zeitpunkten 150 µl Überstand entnommen und bei -20°C gelagert.

### HPLC-Messungen

Die HPLC-Messungen wurden an einem Agilent 1260 Infinity II-System (Santa Clara, USA) durchgeführt. Die LC war mit einem Multisampler (G7167A), einem DAD (Diodenarray-Detektor) für UV und den sichtbaren Lichtbereich und einer Zorbax Eclipse Plus C18-Säule (4,6 mm × 150 mm, 5 µm, Agilent, Santa Clara, USA) ausgestattet. Die Messung der Adipinsäure erfolgte mit einer Gradienten Methode bestehend aus wässrigem Laufmittel (0,085 % v/v Phosphorsäure in demineralisiertem Wasser) und 99,8 % Acetonitril bei 35°C Säulentemperatur (Tabelle 3). Die Flussrate des Laufmittels betrug 1,5 ml/min und es wurden 5 µl Probe injiziert. Die Daten wurden mit der Software Chromeleon Chromatography Data System 7.3.1 (Thermo Scientific, Waltham, USA) analysiert.

**Tabelle 3: HPLC-Methode mit Laufmittelgradient für Adipinsäure-Quantifizierung.**

| Zeit (min) | 0,085 % Phosphorsäure in dest. Wasser (%) | 99,8 % Acetonitril (%) |
|---|---|---|
| 0 | 95 | 5 |
| 0,5 | 95 | 5 |
| 4,5 | 75 | 25 |
| 8,5 | 5 | 95 |
| 10,5 | 5 | 95 |
| 11,5 | 95 | 5 |
| 14 | 95 | 5 |

### TPU-Bioabbaubarkeitsstudien im biologischen System (nach OECD 301 F)

Für die Bioabbaubarkeitsstudien wurde nach der OECD (Organisation für wirtschaftliche Zusammenarbeit und Entwicklung) Richtlinie 301 F zur "manometrischen respiratorischen Bestimmung der Bioabbaubarkeit von chemischen Testmaterialien" vorgegangen. Es wurde das System BD 600 von Lovibond verwendet. Für die Durchführung der Bioabbaubarkeitsstudie wurde eine vorbestimmte Menge der zu testenden Substanz im Testmedium (Minimalsalzmedium mit Klärschlamm) gelöst. Die Lösung wurde in einer geschlossenen Flasche im Dunkeln unter Rühren bei konstanter Temperatur von 20°C für 90 Tage inkubiert. Der biologische Abbau der Testsubstanz wurde durch Messung des biochemischen Sauerstoffbedarfs (BOD = biochemical oxygen demand) gemessen. Die folgenden Schritte wurden dafür durchgeführt:
1. Berechnung der Menge an benötigter Testsubstanz (Anhang IV in OECD 301)
   a) Theoretischer Sauerstoffverbrauch (ThOD = theoretical oxygen demand):
      Testsubstanz: C_{c}HₕCl_{cl}NₙNaₙₐOₒPₚSₛ $ThoD \left(\frac{mg {O}_{2}}{mg Testsubstanz}\right) = \frac{16 \left[2C+0,5 \left(H-Cl-3N\right)+3S+2,5P+0,5 Na-O\right]}{MW}$ Diese Berechnung impliziert, dass C zu CO₂, H zu H₂O, P zu P₂O₅ und Na zu Na₂O mineralisiert wird. Das Halogen (CI = Chlor) wird als Wasserstoffhalogenid und Stickstoff als Ammoniak eliminiert. Hinweis: Ein Nitrifikationshemmer kann zu den Proben hinzugefügt werden (siehe Schritt 7), um sicherzustellen, dass Stickstoff als Ammoniak eliminiert wird und keine Nitrifikation stattfindet.
   b) Berechnung der benötigten Menge der Testsubstanz unter Berücksichtigung des Volumens des Testmediums (157 ml) und des maximalen BOD-Werts im Messbereich des verwendeten Equipments (400 mg/I): $mg Testsubstanz = \frac{400 mg {O}_{2}}{1 I Wasser} ∗ 0 , 157 I Wasser ∗ \frac{1 mg Testsubstanz}{ThOD mg {O}_{2}}$
2. Der in der Abbaustudie verwendete Klärschlamm wird für mindestens 24 Stunden mit Druckluft bei konstanter Temperatur (23 °C) durchströmt.
3. Für das Minimalsalzmedium wurden die Angaben in OECD 301 A, Abschnitt 5 befolgt
4. Zusammensetzung von 1 I Testmedium:
   a. 10 ml konzentrierter Klärschlamm (Ablagerung am Boden des Klärschlamm Containers)
   b. 15 ml verdünnter Klärschlamm (Überstand des Klärschlamm Containers)
   c. 970 g demineralisiertes Wasser
   d. 1 ml der Salz- und Spurenelementlösungen A-D aus OECD 301 A, Abschnitt 5
5. Das Testmedium wurde bei 20°C gesättigt, indem es 20 Minuten lang mit sauberer Druckluft unter Rühren belüftet wurde.
6. Die Abbaubarkeitsstudie wurde in Duplikaten durchgeführt, wobei Natriumacetat und eine Leerprobe (ohne Testsubtanz oder Natriumacetat) als Referenzen mitgemessen wurden.
7. Zusammensetzung des Reaktionsansatzes: Berechnete Menge der Testsubstanz zugegeben in 157 ml Testmedium und Zugabe von 5 Tropfen Nitrifikationshemmer (Allylthiourea)
8. Berechnung des BOD bei vollständiger Abbaubarkeit nach folgender Formel: $BOD bei vollständigem Abbau \left(\frac{mg {O}_{2}}{I}\right) = mg Testsubstanz ∗ \frac{ThOD mg {O}_{2}}{1 mg Testsubstanz} ∗ \frac{1}{0 , 157 I Wasser}$
10. Berechnung der Bioabbaubarkeitsrate: $Bioabbaubarkeit \left(%\right) = \frac{BOD gemessen \left(\frac{mg {O}_{2}}{I}\right)}{BOD bei vollständigen Abbau \left(\frac{mg {O}_{2}}{I}\right)} ∗ 100$

### Konstruktion des phylogenetischen Baums und Konsensussequenz

Für die Analyse der Sequenzidentitäten wurde ein Sequenzalignment mit der Software Geneious Prime^{®} 2023.2.1 mit dem Clustal Omega 1.2.2 Algorithmus durchgeführt.

Die phylogenetische Analyse erfolgte in der MEGA11-Software unter Anwendung der Maximum-Likelihood-Methode.

Der phylogenetische Baum wurde mit dem statistischen Bootstrap-Verfahren generiert, wobei die Anzahl an Replikaten bei 100 lag. Dafür wurden die Neighbor-Join- und BioNJ-Algorithmen verwendet, um für die heuristische Suche ursprüngliche Bäume auf einer Matrix von paarweisen Distanzen automatisch zu erzeugen. Die Distanzen wurden wiederum mit dem Jones-TaylorThornton (JTT)-Modell und der Topologie mit dem überlegenen Log-Likelihood-Wert geschätzt. Die phylogenetische Analyse umfasste 10 Aminosäuresequenzen. Die Sequenz der alkalischen Phosphatase aus *E. coli* wurde als Outgroup verwendet.

### Ergebnisse

### Abbau der lösemittelbasiert hergestellten TPU/Enzym Zusammensetzung unter Laborbedingungen im wässrigen System

Die in Tabelle 1 aufgelisteten Enzyme wurden in TPU 1 über ein lösemittelbasiertes Verfahren eingearbeitet. Die Hydrolyseaktivität der Enzyme nach Einarbeitung wurde unter Laborbedingungen im wässrigen Puffersystem anhand der Monomerfreisetzung (Adipinsäure) gemessen. Die Ergebnisse dazu sind in Tabelle 4 zu finden. Novozym^{®} 51032, zeigte die stärkste Hydrolyse von TPU 1 nach Einarbeitung. Dieses Enzym hat vermutlich eine Aminosäuresequenz wie durch SEQ ID NO.: 5 definiert. Weiterhin zeigten alle anderen getesteten Cutinasen ebenfalls eine Hydrolyse von TPU 1 nach Einarbeitung, die sich signifikant von der Hintergrundaktivität der Leervektorkontrolle abhebt. Jegliche Adipinsäurefreisetzung die mehr als 0,25 % betrug wurde als hydrolytische Aktivität durch die eingearbeiteten Cutinasen gewertet. Alle gemessenen Adipinsäurewerte wurden durch die Blankwerte der Autohydrolyse und Hintergrundaktivität durch die Leervektorkontrolle korrigiert. Somit spiegeln die in Tabelle 4 aufgelisteten Werte die Adipinsäuremenge wider, die einzig durch die hydrolytische Aktivität der eingearbeiteten Cutinasen freigesetzt wurde. In allen Fällen führte eine höhere Enzymkonzentration zu einer höheren Adipinsäurefreisetzung. Der Umsatz zu Adipinsäure ist in Tabelle 4 in % angegeben und bezieht sich auf die Menge, die vom Gesamtanteil der in der TPU-Probe enthaltenen Mengen Adipinsäure freigesetzt wurde.

**Tabelle 4: Quantifizierung der Adipinsäurefreisetzung aus TPU 1 Filmen nach 21 Tagen Inkubationszeit bei 37°C. Alle Filme enthielten durch ein lösemittelbasiertes Verfahren eingearbeitete Enzyme in einer Menge von 0,3 oder 0,5 Gew.-%.**

| **SEQ ID NO.:** | **Enzym** | **eingearbeitete Enzymmenge [Gew.-%]** | **Hydrolyse von TPU 1 nach LM-basierter Einarbeitung** | **Umsatz zu Adipinsäure [%]** |
|---|---|---|---|---|
| 5** | Novozym^{®} 51032 | 0,3 | + | 3,21**** |
| 5** | Novozym^{®} 51032 | 0,5 | + | 4,53**** |
| 2 | ThcCut1 | 0,3 | + | 2,77*** |
| 2 | ThcCut1 | 0,5 | + | 2,93*** |
| 3 | ThcCut1-ACCG | 0,3 | + | 1,06*** |
| 3 | ThcCut1-ACCG | 0,5 | + | 2,8*** |
| 4 | SvCut190*SS | 0,3 | + | 2,02*** |
| 4 | SvCut190*SS | 0,5 | + | 2,3*** |
| 10 | LCC-ICCG | 0,3 | + | 1,63*** |
| 10 | LCC-ICCG | 0,5 | + | 3,05*** |
| 8 | LCC | 0,5 | + | 1,6*** |
| 9 | LCC-WCCG | 0,5 | + | 1,79*** |
| 7 | TfCut2 | 0,5 | + | 0,54*** |
| 6 | PesH1 | 0,5 | + | 1,83*** |
| - | pET26b_LV | 0,3 | - | -**** |
| - | pET26b_LV | 0,5 | - | 0,25**** |
| - | - | - | - | - |

| | | | | |
|---|---|---|---|---|
| ** Angabe basiert auf Literaturrecherche *** Wert wurde um Leervektor- und Negativkontrolle korrigiert **** Wert wurde um Negativkontrolle korrigiert | | | | |

### Abbau der durch Schmelz-Extrusion hergestellten TPU/Enzym Zusammensetzung unter in vitro Laborbedingungen

Die Restaktivität, der durch Schmelz-Extrusion in TPU 1 und 2 eingearbeiteten Enzyme, wurde unter Laborbedingungen im wässrigen System getestet und über den Nachweis des durch den Abbau freigesetzten Monomers Adipinsäure, evaluiert. Für alle in Tabelle 5 aufgelisteten Enzyme konnte eine hydrolytische Aktivität nach Einarbeitung in das TPU 2 durch Adipinsäurefreisetzung nachgewiesen werden, während in der Leervektorkontrolle (pET26b_LV) keine Monomerfreisetzung nachgewiesen wurde. Die Kombination aus Calciumcarbonat und Novozym^{®} 51032 verbesserte die Hydrolyse von TPU 2 im Vergleich zum Ansatz, welcher nur Novozym^{®} 51032, aber kein Calciumcarbonat enthielt. Die Einarbeitung in TPU 1 erfolgte ausschließlich mit den Enzymen Novozym^{®} 51032 und SvCut190*SS, wobei für beide Enzyme hydrolytische Aktivität nach Einarbeitung über Schmelzextrusion nachgewiesen wurde, während in der Leervektorkontrolle (pET26b_LV) keine Monomerfreisetzung nachgewiesen wurde (Tabelle 6).

**Tabelle 5: Quantifizierung der Adipinsäurefreisetzung aus TPU 2 mit über Schmelzextrusion eingearbeiteten Cutinasen nach 21 Tagen Inkubationszeit bei 37°C. Alle TPU-Proben enthielten 0,3 Gew.-% Enzym- bzw. Leervektor-Lyophilisat und wurden bei einer Schmelztemperatur von 150°C eingearbeitet. Es wurden 20 Gew.-% Calciumcarbonat zusammen mit Novozym^{®} 51032 eingearbeitet. Die Werte sind als Mittelwerte von Triplikaten angegeben mit Ausnahme von Novozym^{®} 51032 + Calciumcarbonat, welche als einfache Probe gemessen wurde.**

| **SEQ ID NO.:** | **Enzym** | **Hydrolyse von TPU 2 nach Hitzebasierter Einarbeitung** | **Umsatz zu Adipinsäure aus TPU 2 [%]** |
|---|---|---|---|
| 5** | Novozym^{®} 51032 | + | 36,5**** |
| 5** | Novozym^{®} 51032 + Calciumcarbonat | + | 46,82**** |
| 3 | ThcCut1-ACCG | + | 1,6*** |
| 4 | SvCut190*SS | + | 1,3*** |
| 10 | LCC-ICCG | + | 0,7*** |
| - | pET26b_LV | - | 0,9**** |

| | | | |
|---|---|---|---|
| ** Angabe basiert auf Literaturrecherche *** Wert wurde um Leervektor- und Negativkontrolle korrigiert **** Wert wurde um Negativkontrolle korrigiert | | | |

**Tabelle 6: Quantifizierung der Adipinsäurefreisetzung aus TPU 1 mit über Schmelzextrusion eingearbeiteten Cutinasen nach 21 Tagen Inkubationszeit bei 37°C. Alle TPU-Proben enthielten 0,3 Gew.-% Enzym- bzw. Leervektor-Lyophilisat und wurden bei einer Schmelztemperatur von 190°C bzw. der Leervektor bei 195°C eingearbeitet. Die Werte sind als Mittelwerte von Triplikaten angegeben.**

| **SEQ ID NO.:** | **Enzym** | **Hydrolyse von TPU 1 nach Hitzebasierter Einarbeitung** | **Umsatz zu Adipinsäure aus TPU 1 [%]** |
|---|---|---|---|
| 5** | Novozym^{®} 51032 | + | 4,3**** |
| 4 | SvCut190*SS | + | 0,3*** |
| - | pET26b_LV | - | -**** |

| | | | |
|---|---|---|---|
| ** Angabe basiert auf Literaturrecherche *** Wert wurde um Leervektor- und Negativkontrolle korrigiert **** Wert wurde um Negativkontrolle korrigiert | | | |

### Abbau der durch Schmelz-Extrusion hergestellten TPU/Enzym Zusammensetzung unter umweltnahen Bedingungen

Die Biodegradation von TPU 2 wurde gemäß OECD 301 F mit Klärschlamm im *in vivo* System getestet. Die Ergebnisse der Biodegradationsraten nach 90 Tagen Inkubationszeit sind in Tabelle 7 zusammengefasst. Die TPU-Kombination mit Novozym^{®} 51032 zeigte die höchste Biodegradationsrate, wobei die Kombination aus TPU, Novozym^{®} 51032 und Calciumcarbonat eine zusätzliche Verbesserung bewirkte. Für die Leervektorkontrolle wurde die im Vergleich zu allen Kombinationen aus TPU und Enzym, geringste Biodegradationsrate gemessen. Zusätzlich wurde eine TPU-Kombination getestet, die enzymatisch inaktives Protein enthielt. Diese zeigte ebenfalls keine signifikante Steigerung der Biodegradation. Diese Ergebnisse bestätigten die hydrolytische Aktivität aller in Tabelle 7 aufgelisteten Enzyme, nach Einarbeitung in TPU 2 über Schmelzextrusion, was zu einer verbesserten Bioabbaubarkeit im Vergleich zu TPU-Kombinationen ohne hydrolytisch aktive Enzyme führte.

**Tabelle 7: Biodegradationsraten von TPU 2 mit über Schmelz-Extrusion eingearbeiteten Enzymlyophilisaten bzw. Leervektor Lyophilisat als Referenz mit 0,3 Gew.-% Anteil bezogen auf das TPU Gewicht. Es wurden 20 Gew.-% Calciumcarbonat zusammen mit Novozym^{®} 51032 eingearbeitet. Die Werte sind als Mittelwerte von Duplikaten angegeben.**

| **SEQ ID NO.:** | **Enzym** | **Schmelztemperatur [°C]** | **Biodegradationsrate nach 90 Tagen [%]** |
|---|---|---|---|
| 5** | Novozym^{®} 51032 | 150 | 31,2 |
| 5** | Novozym^{®} 51032 + Calcium Carbonat | 150 | 35,7 |
| 3 | ThcCut1-ACCG | 170 | 10,8 |
| 10 | LCC-ICCG | 170 | 9,9 |
| - | pET26b_LV | 150 | 5,0 |

| | | | |
|---|---|---|---|
| ** Angabe basiert auf Literaturrecherche | | | |

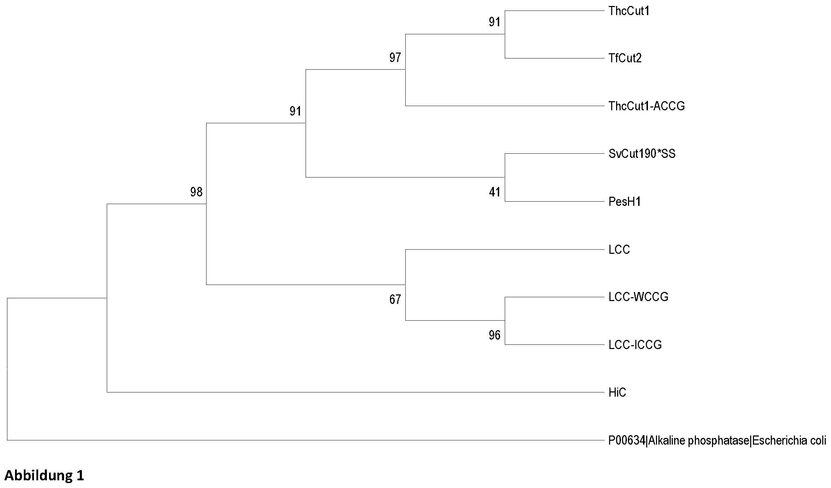

## Patentansprüche

1. Eine Polyesterpolyurethanzusammensetzung enthaltend wenigstens ein Polyesterpolyurethan und
a) wenigstens ein Enzym, das durch eine Aminosäuresequenz gemäß SEQ ID NO.: 1 definiert ist oder eine Variante des vorgenannten Enzyms, die durch eine Aminosäuresequenz mit wenigstens 85 % Identität zu den in SEQ ID NO.: 1 definierten konservierten Aminosäuren definiert ist; oder
b) wenigstens ein Enzym, das durch eine Aminosäuresequenz gemäß SEQ ID NO.: 4 oder 8 definiert ist oder eine Variante eines der vorgenannten Enzyme, die durch eine Aminosäuresequenz mit wenigstens 64 % Identität zu SEQ ID NO.: 4 oder wenigstens 85 % Sequenzidentität zu SEQ ID NO.: 8 definiert ist.

2. Die Polyesterpolyurethanzusammensetzung gemäß Anspruch 1, wobei das Polyesterpolyurethan bezogen auf sein Gesamtgewicht zwischen 50 und 70 Gew.-% Polyesterpolyol enthält.

3. Die Polyesterpolyurethanzusammensetzung gemäß Anspruch 1 oder 2, wobei das Polyesterpolyurethan ein zahlenmittleres Molekulargewicht zwischen 500 g/mol und 4.000 g/mol aufweist.

4. Die Polyesterpolyurethanzusammensetzung gemäß eines der Ansprüche 1 bis 3, wobei das Polyesterpolyurethan ein thermoplastisches Polyesterpolyurethan ist.

5. Die Polyesterpolyurethanzusammensetzung gemäß eines der Ansprüche 1 bis 4, wobei die Variante des durch SEQ ID NO.: 1 definierten Enzyms durch eine Aminosäuresequenz gekennzeichnet ist, die wenigstens 95 % Identität zu den in SEQ ID NO.: 1 definierten konservierten Aminosäuren aufweist.

6. Die Polyesterpolyurethanzusammensetzung gemäß eines der Ansprüche 1 bis 5, zusätzlich enthaltend einen Säurefänger.

7. Die Polyesterpolyurethanzusammensetzung gemäß Anspruch 6, wobei der Säurefänger ein anorganisches Salz ist, ausgewählt aus der Gruppe bestehend aus Carbonaten, Hydrogencarbonaten, Phosphaten und Hydroxiden.

8. Ein Polymerprodukt, das die Polyurethanzusammensetzung wie durch einen der Ansprüche 1 definiert enthält.

9. Verwendung eines Enzyms, das
(i) durch eine Aminosäuresequenz gemäß SEQ ID NO.: 1 definiert ist oder eine Variante des vorgenannten Enzyms, die durch eine Aminosäuresequenz mit wenigstens 85 % Identität zu den in SEQ ID NO.: 1 definierten konservierten Aminosäuren definiert ist; oder
(ii) durch eine Aminosäuresequenz gemäß SEQ ID NO.: 4 oder 8 definiert ist oder eine Variante eines der vorgenannten Enzyme, die durch eine Aminosäuresequenz mit wenigstens 64 % Identität zu SEQ ID NO.: 4 oder wenigstens 85 % Sequenzidentität zu SEQ ID NO.: 8 definiert ist;
Zur Erhöhung der biologischen Abbaubarkeit einer Polyesterpolyurethanzusammensetzung.

10. Ein Verfahren zur Herstellung einer Polyesterpolyurethanzusammensetzung enthaltend die die Schritte
a) Bereitstellung wenigstens eines Polyesterpolyurethans und wenigstens eines Enzyms, das
(i) durch eine Aminosäuresequenz gemäß SEQ ID NO.: 1 definiert ist oder eine Variante des vorgenannten Enzyms, die durch eine Aminosäuresequenz mit wenigstens 85 % Identität zu den in SEQ ID NO.: 1 definierten konservierten Aminosäuren definiert ist; oder
(ii) durch eine Aminosäuresequenz gemäß SEQ ID NO.: 4 oder 8 definiert ist oder eine Variante eines der vorgenannten Enzyme, die durch eine Aminosäuresequenz mit wenigstens 64 % Identität zu SEQ ID NO.: 4 oder wenigstens 85 % Sequenzidentität zu SEQ ID NO.: 8 definiert ist;
b) Vermischen des wenigstens einen Polyesterpolurethans mit dem wenigstens einen Enzym, wobei das wenigstens eine Polyesterpolyurethan entweder als Schmelze oder in einem geeigneten Lösungsmittel gelöst vorliegt; und
c) Aushärten der Polyesterpolyurethanzusammensetzung durch Abkühlung der Schmelze oder durch Entfernung des Lösungsmittels.
